# EUROPÄISCHE PATENTSCHRIFT

(11) **EP 3 717 039 B1**
(45) Veröffentlichungstag und Bekanntmachung des Hinweises auf die Patenterteilung: **10.11.2021**
(21) Anmeldenummer: 18815128.6
(22) Anmeldetag: 28.11.2018
(51) Int. Cl.: A61M 1/16

(54) **DIALYSEGERÄT MIT VERWECHSLUNGSSICHERUNG FÜR HYDRAULIKANSCHLÜSSE**
DIALYSIS APPLIANCE WITH POKA-YOKE FOR HYDRAULIC PORTS
APPAREIL DE DIALYSE AVEC SÉCURITÉ ANTI-CONFUSION POUR RACCORDEMENTS HYDRAULIQUES

(30) Priorität: 28.11.2017 DE 102017128080
(43) Veröffentlichungstag der Anmeldung: 07.10.2020
(73) Patentinhaber: Fresenius Medical Care Deutschland GmbH, 61352 Bad Homburg (DE)
(72) Erfinder: HÄCKER, Jürgen, 61267 Neu-Ansprach (DE); SCHEUNERT, Peter, 61381 Friedrichsdorf (DE); BOND, Oliver, 97520 Heidenfeld (DE); BREHM, Winfried, 97461 Hofheim (DE); KALSER, Martin, 97437 Haßfurt (DE)
(74) Vertreter: Herrmann, Uwe
(86) Internationale Anmeldenummer: PCT/EP2018/082887
(87) Internationale Veröffentlichungsnummer: WO 2019/106039

(56) Entgegenhaltungen:
- DE-A1- 4 303 372
- US-A- 5 589 070
- US-A1- 2016 243 298

## Beschreibung

Die Erfindung betrifft ein Dialysegerät mit einem Zulaufanschluss zur Zuleitung frischer Dialysierflüssigkeit zum Dialysator, einem Rücklaufanschluss zur Ableitung verbrauchten Dialysats vom Dialysator und zwei je einem Anschluss zugeordneten Parkstellen, wobei eine Verwechslungssicherung vorgesehen ist, die eine Lagerung des Zulaufanschluss und des Rücklaufanschlusses an der jeweils zugehörigen Parkstelle sicherstellt. Ein Dialysegerät ist beispielsweise aus der US2016/243298A1 oder dem patent US5589070A bekannt. Weiterhin betrifft die DE4303372A1 einen Beutel zur Aufnahme von festem oder flüssigem Konzentrat für die Herstellung von Dialysierflüssigkeit mittels einer Dialysemaschine mit einem Beutelkörper und mit mindestens einer Öffnung sowie eine Steckerverbindung zur Verwendung mit einem solchen Beutel.

Die Dialysatorschnittstelle ist eine fluidische Schnittstelle eines Dialysegeräts, an die erhöhte hygienische Anforderungen gestellt werden. Der Dialysator verfügt über zwei dialysatseitige Hydraulikanschlüsse. Einer dient als Zulauf für frische Dialysierflüssigkeit, der andere zur Abführung des verbrauchten Dialysats. Verbrauchtes Dialysat ist potentiell infektiös. Beim Abrüsten nach einer Behandlung gelangt Dialysat auf die Parkstellen bzw. -anschlüsse des Geräts. Über eine Farbkodierung wird derzeit angegeben, auf welche Parkstelle welcher Konnektor zu stecken ist (beispielsweise rot zu rot und blau zu blau). Tritt hier aufgrund fehlerhafter Handhabung eine Vertauschung auf, besteht die Gefahr, dass die Zulaufkupplung kontaminiert wird. Bei einer anschließenden Behandlung könnten dann pathogene Organismen auf die Hydraulikseite des Dialysators gelangen.

Aufgabe der Erfindung ist es, ein Dialysegerät bereitzustellen, mit der eine solche Verwechslung sicher ausgeschlossen werden kann.

Die Erfindung wird durch die Merkmale der unabhängigen Ansprüche 1, 9 und 10 definiert. Vor diesem Hintergrund betrifft die Erfindung ein Dialysegerät mit zwei dialysatseitigen Hydraulikanschlüssen, an denen je eine Verbindungsleitung mit einer Filterkupplung zur Verbindung mit einem Dialysator angebunden ist, wobei es sich bei den Hydraulikanschlüssen um einen Zulaufanschluss zur Zuleitung frischer Dialysierflüssigkeit zum Dialysator und um einen Rücklaufanschluss zur Ableitung verbrauchten Dialysats vom Dialysator handelt, sowie mit zwei den jeweiligen Hydraulikanschlüssen zugeordneten Parkstellen zum Lagern der Filterkupplungen bei Nichtbetrieb. Es ist vorgesehen, dass die Filterkupplungen beider Hydraulikanschlüsse mit einem Konnektionssensor ausgestattet sind, der eine Konnektierung der Filterkupplung an einen Dialysator erkennt, und/oder dass beide Parkstellen mit einer separaten Abdeckung versehen sind.

Geeignete Abdeckungen umfassen beispielsweise Abdeckklappen oder Schiebedeckel. Die Parkstelle umfasst typischerweise einen Kurzschlussanschluss, mit welchem die Filterkupplung verbunden wird, um das System im Nichtbetrieb spülen zu können.

Erfindungsgemäß ist vorgesehen, dass das Dialysegerät lösbare Verriegelungen zum Sperren der Abdeckungen der Parkstellen aufweist. Beispiele geeigneter Verriegelung umfassen elektromagnetische oder elektromechanische Riegel. In einer Ausführungsform ist vorgesehen, dass das Dialysegerät Abdeckungssensoren zur Erkennung der Stellung der Abdeckungen der Parkstellen aufweist.

In einer Ausführungsform ist vorgesehen, dass das Dialysegerät Aktoren zum Öffnen und Schließen der Abdeckungen der Parkstellen aufweist. Beispiele geeigneter Aktoren umfassen Elektromotoren.

In einer Ausführungsform ist vorgesehen, dass das Dialysegerät eine Signaleinheit zur Ausgabe von Warnsignalen aufweist. Geeignete Warnsignale umfassen akustische und visuelle Warnsignale.

In einer Ausführungsform ist vorgesehen, dass das Dialysegerät eine Steuereinheit aufweist, die mit den Konnektionssensoren und vorzugsweise ferner mit den Verriegelungen, Abdeckungssensoren und/oder Aktoren in Verbindung steht. Die Verbindung kann drahtlos oder drahtgebunden sein.

In einer Ausführungsform ist vorgesehen, dass die Steuereinheit so ausgebildet ist, dass bei Erkennung der Dekonnektierung einer Filterkupplung vom Dialysator die dem betreffenden Hydraulikanschluss zugeordnete Parkstelle freigegeben wird oder bleibt und/oder die dem betreffenden Hydraulikanschluss nicht zugeordnete Parkstelle gesperrt wird oder bleibt. Auf diese Weise kann beim Abrüstvorgang, also beim Trennen des Dialysators und des Dialysegeräts sichergestellt werden, dass die Filterkupplungen an den richtigen Parkstellen gelagert werden.

Die Steuereinheit kann so ausgebildet sein, dass bei Erkennung einer Dekonnektierung der Filterkupplung vom Dialysator die Verriegelung der dem betreffenden Hydraulikanschluss zugeordneten Parkstelle gelöst wird oder bleibt und/oder die Verriegelung der dem betreffenden Hydraulikanschluss nicht zugeordneten Parkstelle gesperrt wird oder bleibt.

Sofern das Dialysegerät Aktoren zum Öffnen und Schließen der Abdeckungen der Parkstellen aufweist, kann die Steuereinheit so ausgebildet sein, dass bei Erkennung einer Dekonnektierung der Filterkupplung vom Dialysator die Abdeckung der dem betreffenden Hydraulikanschluss zugeordneten Parkstelle geöffnet wird oder bleibt und/oder die Abdeckung der dem betreffenden Hydraulikanschluss nicht zugeordneten Parkstelle geschlossen wird oder bleibt.

Sofern das Dialysegerät Abdeckungssensoren zur Erkennung der Stellung der Abdeckungen der Parkstellen aufweist und ferner eine Signaleinheit aufweist, kann die Steuereinheit so ausgebildet sein, dass ein Warnsignal ausgegeben wird, wenn bei Erkennung einer Dekonnektierung der Filterkupplung vom Dialysator die Abdeckung der dem betreffenden Hydraulikanschluss nicht zugeordneten Parkstelle geöffnet wird oder ist.

In einer Ausführungsform ist vorgesehen, dass die Steuereinheit so ausgebildet ist, dass bei Erkennung einer Öffnung der Abdeckung einer Parkstelle die Verriegelung der Abdeckung der anderen Parkstelle gesperrt wird, bis eine Konnektierung derjenigen Filterkupplung an einen Dialysator erkannt wird, deren zugehörige Parkstelle durch Öffnen der Abdeckung freigegeben wurde. Auf diese Weise kann beim Aufrüstvorgang, also beim Verbinden des Dialysators und des Dialysegeräts sichergestellt werden, dass eine Filterkupplung beim Hantieren nicht versehentlich an die falsche Parkstelle zurückgeführt wird.

Vor dem eingengs genannten Hintergrund betrifft die Erfindung ferner ein Verfahren zur Trennung eines Dialysators von einem erfindungsgemäßen Dialysegerät, wobei bei Erkennung der Dekonnektierung einer Filterkupplung vom Dialysator die dem betreffenden Hydraulikanschluss zugeordnete Parkstelle freigegeben wird oder bleibt und/oder die dem betreffenden Hydraulikanschluss nicht zugeordnete Parkstelle gesperrt wird oder bleibt.

Des weiteren betrifft die Erfindung ferner ein Verfahren zur Verbindung eines Dialysators mit einem erfindungsgemäßen Dialysegerät, wobei bei Erkennung einer Öffnung der Abdeckung einer Parkstelle die Verriegelung der Abdeckung der anderen Parkstelle gesperrt wird, bis eine Konnektierung derjenigen Filterkupplung an einen Dialysator erkannt wird, deren zugehörige Parkstelle durch Öffnen der Abdeckung freigegeben wurde.

Weitere Einzelheiten und Vorteile der Erfindung ergeben sich aus dem nachfolgend anhand der Figuren beschriebenen Ausführungsbeispiel. In den Figuren zeigen:

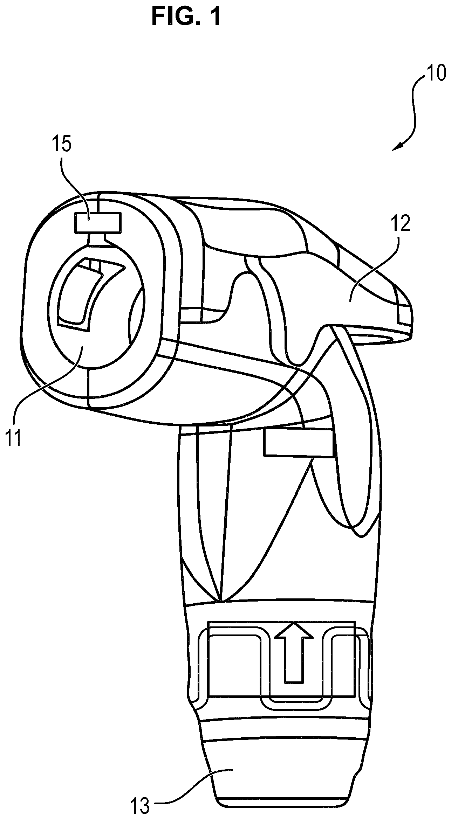

- Figur 1:: eine Filterkupplung eines erfindungsgemäßen Dialysegeräts;

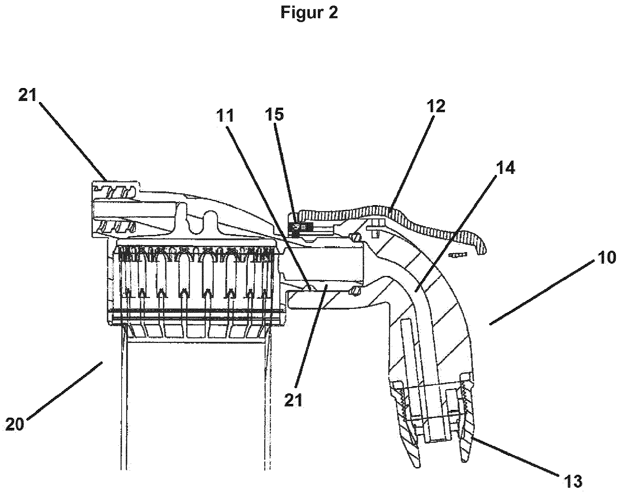

- Figur 2:: die Filterkupplung der Figur 1 in einem am Dialysator angeschlossenen Zustand; und

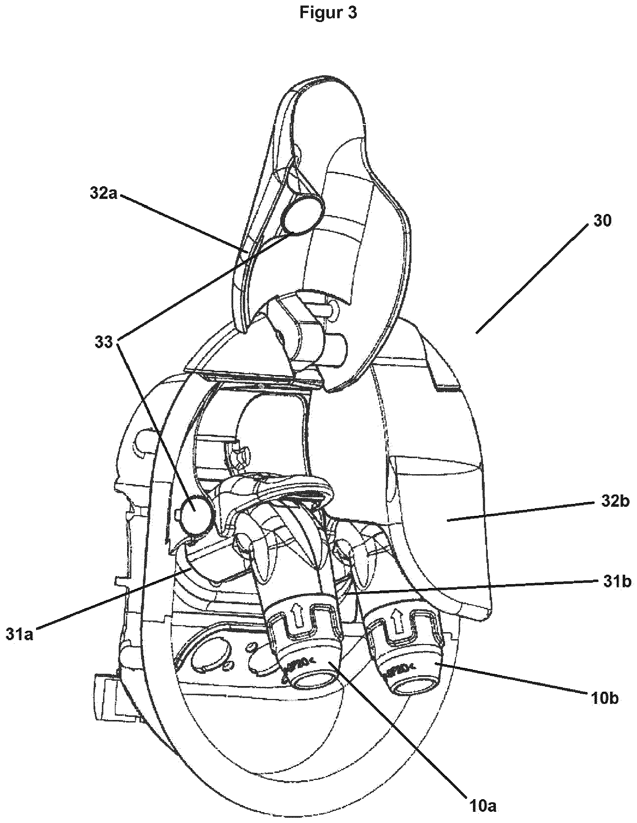

- Figur 3:: eine Parkstation eines erfindungsgemäßen Dialysegeräts.

Die Filterkupplungen 10 des in den Figuren dargestellten Beispiels eines erfindungsgemäßen Dialysegeräts sind dazu ausgebildet, manuell mit Anschlüssen 21 eines Dialysators 20 verbunden zu werden. Das Dialysegerät umfasst zwei derartige Filterkupplungen, eine zur Verbindung mit dem Zulaufanschluss des Dialysators und eine zur Verbindung mit dem Rücklaufanschluss des Dialysators.

Die Filterkupplungen 10 umfassen eine weibliche Aufnahme 11, in die ein männlicher Anschluss 21 eines Dialysators 20 eingesteckt werden kann. Ein Betätigungshebel 12 dient zum Lösen und Festsetzen der Aufnahme 11 am Anschluss 21. An der gegenüberliegenden Seite der Filterkupplungen 10 befindet sich ein Anbindungsbereich 13 für einen Flüssigkeitsschlauch. Zwischen der Aufnahme 11 und dem Anbindungsbereich 13 ist ein Fluidkanal 14 gebildet. Kennzeichnend für die vorliegende Erfindung ist der in der Aufnahme 11 sitzende Konnektionssensor 15, der eine Verbindung der Filterkupplung 10 mit dem Anschluss 21 erkennen kann. Beispielsweise handelt es sich dabei um einen Berührungssensor. Er kann drahtlos oder anhand eines nicht dargestellten Verbindungsdrahtes mit der Steuereinheit des Dialysegeräts in Verbindung stehen.

An einer Park- bzw. Kurzschlussstation 30 des Dialysegeräts befinden sich Kurzschlussanschlüsse 31a und 31b für beide Filterkupplungen 10a und 10b des Dialysegeräts. Jeder dieser Kurzschlussanschlüsse 31a und 31b ist mit einer separaten Abdeckklappe 32a bzw. 32b überdeckt. Die Abdeckungssensoren 33a und 33b dienen der Erkennung der Stellung der jeweiligen Abdeckklappe 32a bzw. 32b. Das Dialysegerät umfasst ferner nicht näher dargestellte elektromagnetische Riegel zum Sperren der Abdeckklappen 32a und 32b sowie nicht näher dargestellte elektrische Aktoren zum Öffnen und Schließen der Abdeckklappen 32a und 32b. Die Abdeckungssensoren 33a und 33b stehen ebenso wie die Riegel und Aktoren mit der Steuereinheit des Gerätes in Verbindung. Die Steuereinheit steht mit einer Signaleinrichtung in Verbindung, die Warnsignale ausgeben kann.

Somit sind in einem erfindungsgemäßen Dialysegerät die Filterkupplungen 10 mit je einem Konnektionssensor 15 ausgestattet, welcher die Konnektionsstellung, d.h. die Verbindung mit einem Anschluss eines Dialysators 20 erkennt. Ferner wird die Kurzschlussstation 30 aufgeteilt und jeder Kurzschlussanschluss 31a und 31b mit einer separaten Abdeckklappe 32a bzw. 32b inkl. Abdeckungssensor 33 versehen. Die Kurzschlussanschlüsse 31a und 31b, auf welche die Filterkupplungen 10a bzw. 10b bei Nicht-Betrieb oder während des Reinigungsprogramms deponiert werden, sind also mit je einer eignen mechanischen Abdeckklappe 32a bzw. 32b ausgestattet, welche einzeln überwacht betätigt werden kann.

Der Abrüstvorgang, also das Trennen des Dialysators 20 von dem Dialysegerät kann folgendermaßen erfolgen: Eine der Filterkupplungen 10 wird vom Anwender einzeln vom zugeordneten Anschluss 21 des Dialysators gelöst. Der Konnektionssensor 15 erkennt, welche der beiden Kupplungen 10 dekonnektiert wurde und gibt den dazugehörigen Kurzschlussanschluss 31 frei bzw. sperrt den nicht dazugehörigen Kurzschlussanschluss 31. Freigabe kann bedeuten, dass (a) eine Verriegelung der Klappe 32 aufgehoben wird, (b) die Klappe 32 automatisch mittels Aktor geöffnet wird, oder (c) kein Warnsignal ausgegeben wird, wenn die Klappe 32 manuell geöffnet wird. Sperrung kann bedeuten, dass (a) die Klappe 32 verriegelt wird, oder (b) ein Warnsignal ausgegeben wird, wenn die Klappe 32 manuell geöffnet wird.

Der Aufrüstvorgang, also das Verbinden des Dialysators und des Dialysegeräts kann folgendermaßen erfolgen: Eine Klappe 32 wird vom Anwender geöffnet. Der Abdeckungssensor 33 erkennt welche Klappe 32 geöffnet wird und sperrt, im obigen Sinne, die andere Klappe.

## Patentansprüche

1. Dialysegerät mit zwei dialysatseitigen Hydraulikanschlüssen, an denen je eine Verbindungsleitung mit einer Filterkupplung (10) zur Verbindung mit einem Dialysator (20) angebunden ist, wobei es sich bei den Hydraulikanschlüssen um einen Zulaufanschluss zur Zuleitung frischer Dialysierflüssigkeit zum Dialysator (20) und um einen Rücklaufanschluss zur Ableitung verbrauchten Dialysats vom Dialysator (20) handelt, sowie mit zwei den jeweiligen Hydraulikanschlüssen zugeordneten Parkstellen (31a, 31b) zum Lagern der Filterkupplungen (10) bei Nichtbetrieb,
**dadurch gekennzeichnet, dass**
beide Parkstellen (31a, 31b) mit einer separaten Abdeckung (32a, 32b) versehen sind und das Dialysegerät lösbare Verriegelungen zum Sperren der Abdeckungen (32a, 32b) der Parkstellen aufweist.

2. Dialysegerät nach Anspruch 1, **dadurch gekennzeichnet, dass** die Filterkupplungen (10) beider Hydraulikanschlüsse mit einem Konnektionssensor (15) ausgestattet sind, der eine Konnektierung der Filterkupplung (10) an einen Dialysator (20) erkennt.

3. Dialysegerät nach einem der vorhergehenden Ansprüche, **dadurch gekennzeichnet, dass** das Dialysegerät Abdeckungssensoren (33) zur Erkennung der Stellung der Abdeckungen (32a, 32b) der Parkstellen aufweist.

4. Dialysegerät nach einem der vorhergehenden Ansprüche, **dadurch gekennzeichnet, dass** das Dialysegerät Aktoren zum öffnen und Schließen der Abdeckungen (32a, 32b) der Parkstellen aufweist.

5. Dialysegerät nach einem der vorhergehenden Ansprüche, **dadurch gekennzeichnet, dass** das Dialysegerät eine Signaleinheit zur Ausgabe von Warnsignalen aufweist.

6. Dialysegerät nach einem der vorhergehenden Ansprüche, zumindest jedoch fortgebildet mit den Merkmalen des Anspruchs 2, **dadurch gekennzeichnet, dass** das Dialysegerät eine Steuereinheit aufweist, die mit den Konnektionssensoren (15) und vorzugsweise ferner mit den Verriegelungen, Abdeckungssensoren (33) und/oder Aktoren in Verbindung steht.

7. Dialysegerät nach Anspruch 6 und fortgebildet mit den Merkmalen des Anspruchs 1, **dadurch gekennzeichnet, dass** die Steuereinheit so ausgebildet ist, dass bei Erkennung der Dekonnektierung einer Filterkupplung (10) vom Dialysator (20) die dem betreffenden Hydraulikanschluss zugeordnete Parkstelle (31a, 31b) freigegeben wird oder bleibt und/oder die dem betreffenden Hydraulikanschluss nicht zugeordnete Parkstelle (31a, 31b) gesperrt wird oder bleibt.

8. Dialysegerät nach Ansprüchen 2, 1, 3 und 6, **dadurch gekennzeichnet, dass** die Steuereinheit so ausgebildet ist, dass bei Erkennung einer Öffnung der Abdeckung (32a, 32b) einer Parkstelle (31a, 31b) die Verriegelung der Abdeckung (32a, 32b) der anderen Parkstelle (31a, 31b) gesperrt wird, bis eine Konnektierung derjenigen Filterkupplung (10) an einen Dialysator (20) erkannt wird, deren zugehörige Parkstelle (31a, 31b) durch Öffnen der Abdeckung (32a, 32b) freigegeben wurde.

9. Verfahren zur Trennung eines Dialysators (20) von einem Dialysegerät nach einem der vorhergehenden Ansprüche, zumindest jedoch fortgebildet mit den Merkmalen des Anspruchs 2,
**dadurch gekennzeichnet,**
**dass** bei Erkennung der Dekonnektierung einer Filterkupplung (10) vom Dialysator (20) die dem betreffenden Hydraulikanschluss zugeordnete Parkstelle (31a, 31b) freigegeben wird oder bleibt und/oder die dem betreffenden Hydraulikanschluss nicht zugeordnete Parkstelle (31a, 31b) gesperrt wird oder bleibt.

10. Verfahren zur Verbindung eines Dialysators (20) mit einem Dialysegerät nach Ansprüchen 2, 1, 3 und 6,
**dadurch gekennzeichnet,**
**dass** bei Erkennung einer Öffnung der Abdeckung (32a, 32b) einer Parkstelle (31a, 31b) die Verriegelung der Abdeckung (32a, 32b) der anderen Parkstelle (31a, 31b) gesperrt wird, bis eine Konnektierung derjenigen Filterkupplung (10) an einen Dialysator (20) erkannt wird, deren zugehörige Parkstelle (31a, 31b) durch Öffnen der Abdeckung (32a, 32b) freigegeben wurde.

## Claims

1. A dialysis machine having two hydraulic connectors at the dialyzate side to which a respective connection line having a filter coupling (10) is connected for connection to a dialyzer (20), with the hydraulic connectors being an inflow connector for feeding fresh dialysis liquid to the dialyzer (20) and a backflow connector for draining consumed dialyzate from the dialyzer (20), and having two parking positions (31a, 31b) associated with the respective hydraulic connectors for storing the filter couplings (10) when not in operation,
**characterized in that**
both parking positions (31a, 31b) are provided with separate covers (32a, 32b) and the dialysis machine has releasable latching mechanisms for blocking the covers (32a, 32b) of the parking positions.

2. A dialysis machine in accordance with claim 1, **characterized in that** the filter couplings (10) of both hydraulic connectors are provided with a connection sensor (15) that recognizes a connection of the filter coupling (10) to a dialyzer (20).

3. A dialysis machine in accordance with one of the preceding claims, **characterized in that** the dialysis machine has cover sensors (33) for recognizing the position of the covers (32a, 32b) of the parking positions.

4. A dialysis machine in accordance with one of the preceding claims, **characterized in that** the dialysis machine has actuators for opening and closing the covers (32a, 32b) of the parking positions.

5. A dialysis machine in accordance with one of the preceding claims, **characterized in that** the dialysis machine has a signal unit for outputting warning signals.

6. A dialysis machine in accordance with one of the preceding claims, however, at least further developed in accordance with the feaures of claim 2, **characterized in that** the dialysis machine has a control unit that is connected to the connection sensors (15) and is preferably further connected to the latching mechanisms, cover sensors (33), and/or actuators.

7. A dialysis machine in accordance with claim 6 and further developed in accordance with the features of claim 1, **characterized in that** the control unit is configured such that when the disconnecting of a filter coupling (10) from the dialyzer (20) is recognized, the parking position (31a, 31b) associated with the respective hydraulic connector is or remains released and/or the parking position (31a, 31b) not associated with the respective hydraulic connector is or remains blocked.

8. A dialysis machine in accordance with claims 2, 1, 3, and 6, **characterized in that** the control unit is configured such that when an opening of the cover (32a, 32b) of a parking position (31a, 31b) is recognized, the latching mechanism of the cover (32a, 32b) of the other parking position (31a, 31b) is blocked until a connecting of that filter coupling (10) to a dialyzer (20) is recognized whose associated parking position (31a, 31b) was released by opening the cover (32a, 32b).

9. A method of separating a dialyzer (20) from a dialysis machine in accordance with one of the preceding claims, however, at least further developed in accordance with the features of claim 2,
**characterized in that**
when the disconnecting of a filter coupling (10) from the dialyzer (20) is recognized, the parking position (31a, 31b) associated with the respective hydraulic connector is or remains released and/or the parking position (31a, 31b) not associated with the respective hydraulic connector is or remains blocked.

10. A method of connecting a dialyzer (20) to a dialysis machine in accordance with claims 2, 1, 3, and 6,
**characterized in that**
when an opening of the cover (32a, 32b) of a parking position (31a, 31b) is recognized, the latching mechanism of the cover (32a, 32b) of the other parking position (31a, 31b) is blocked until a connecting of that filter coupling (10) to a dialyzer (20) is recognized whose associated parking position (31a, 31b) was released by opening the cover (32a, 32b).

## Revendications

1. Appareil de dialyse comprenant deux raccordements hydrauliques côté dialysat, auxquels est respectivement reliée une conduite de liaison dotée d'un couplage à filtre (10) pour la liaison avec un dialyseur (20), les raccordements hydrauliques étant un raccordement d'admission pour l'alimentation de fluide de dialyse frais vers le dialyseur (20) et un raccordement de retour pour l'évacuation de dialysat usagé depuis le dialyseur (20), ainsi que deux points de rangement (31a, 31b) associés aux raccordements hydrauliques respectifs pour loger les couplages à filtre (10) dans l'état hors fonctionnement,
**caractérisé en ce que**
les deux points de rangement (31a, 31b) sont pourvus d'un élément de recouvrement (32a, 32b) séparé et l'appareil de dialyse comporte des mécanismes de verrouillage libérables pour bloquer les éléments de recouvrement (32a, 32b) des points de rangement.

2. Appareil de dialyse selon la revendication 1, **caractérisé en ce que** les couplages à filtre (10) des deux raccordements hydrauliques sont équipés d'un capteur de connexion (15), qui détecte une connexion du couplage à filtre (10) à un dialyseur (20).

3. Appareil de dialyse selon l'une des revendications précédentes, **caractérisé en ce que** l'appareil de dialyse comporte des capteurs de recouvrement (33) pour détecter la position des éléments de recouvrement (32a, 32b) des points de rangement.

4. Appareil de dialyse selon l'une des revendications précédentes, **caractérisé en ce que** l'appareil de dialyse comporte des actionneurs pour ouvrir et fermer les éléments de recouvrement (32a, 32b) des points de rangement.

5. Appareil de dialyse selon l'une des revendications précédentes, **caractérisé en ce que** l'appareil de dialyse comporte une unité de signalisation pour émettre des signaux d'avertissement.

6. Appareil de dialyse selon l'une des revendications précédentes, toutefois au moins perfectionné avec les caractéristiques de la revendication 2, **caractérisé en ce que** l'appareil de dialyse comporte une unité de commande, qui est reliée aux capteurs de connexion (15) et de préférence en outre aux mécanismes de verrouillage, aux capteurs de recouvrement (33) et/ou aux actionneurs.

7. Appareil de dialyse selon la revendication 6 et perfectionné avec les caractéristiques de la revendication 1, **caractérisé en ce que** l'unité de commande est conçue de telle manière que, lors de la détection de la déconnexion d'un couplage à filtre (10) d'avec le dialyseur (20), le point de rangement (31a, 31b) associé au raccordement hydraulique concerné est ou reste libéré et/ou le point de rangement (31a, 31b) non associé au raccordement hydraulique concerné est ou reste bloqué.

8. Appareil de dialyse selon les revendications 2, 1, 3 et 6, **caractérisé en ce que** l'unité de commande est conçue de telle manière que, lors de la détection d'une ouverture de l'élément de recouvrement (32a, 32b) d'un point de rangement (31a, 31b), le mécanisme de verrouillage de l'élément de recouvrement (32a, 32b) de l'autre point de rangement (31a, 31b) est bloqué jusqu'à ce qu'une connexion du couplage à filtre (10), dont le point de rangement (31a, 31b) associé a été libéré par l'ouverture de l'élément de recouvrement (32a, 32b), à un dialyseur (20) soit détectée.

9. Procédé de séparation d'un dialyseur (20) d'avec un appareil de dialyse selon l'une des revendications précédentes, toutefois au moins perfectionné avec les caractéristiques de la revendication 2,
**caractérisé en ce que**,
lors de la détection de la déconnexion d'un couplage à filtre (10) d'avec le dialyseur (20), le point de rangement (31a, 31b) associé au raccordement hydraulique concerné est ou reste libéré et/ou le point de rangement (31a, 31b) non associé au raccordement hydraulique concerné est ou reste bloqué.

10. Procédé de liaison d'un dialyseur (20) à un appareil de dialyse selon les revendications 2, 1, 3 et 6,
**caractérisé en ce que**,
lors de la détection d'une ouverture de l'élément de recouvrement (32a, 32b) d'un point de rangement (31a, 31b), le mécanisme de verrouillage de l'élément de recouvrement (32a, 32b) de l'autre point de rangement (31a, 31b) est bloqué jusqu'à ce qu'une connexion du couplage à filtre (10), dont le point de rangement (31a, 31b) associé a été libéré par l'ouverture de l'élément de recouvrement (32a, 32b), à un dialyseur (20) soit détectée.
